# EUROPEAN PATENT APPLICATION

(11) **EP 3 482 707 A1**
(43) Date of publication of application: **15.05.2019**
(21) Application number: 16908762.4
(22) Date of filing: 11.07.2016
(51) Int. Cl.: A61B 18/12, A61B 17/32

(54) **ENERGY PROCESSING SYSTEM AND METHOD FOR CONTROLLING SAME**

(71) Applicant: Olympus Corporation, Hachioji-shi, Tokyo 192-8507 (JP)
(72) Inventor: HAYASHIDA, Tsuyoshi, Hachioji-shi Tokyo 192-8507 (JP); HONDA, Satoshi, Hachioji-shi Tokyo 192-8507 (JP); TANAKA, Kazue, Hachioji-shi Tokyo 192-8507 (JP)
(74) Representative: Gunzelmann, Rainer
(86) International application number: PCT/JP2016/070442
(87) International publication number: WO 2018/011858

(57) **Abstract**

An energy treatment system and a control method thereof perform treatment by energy supply of ultrasonic energy and high frequency energy in combination, determine that there is a precursor of occurrence of spark when a precursor of occurrence of spark is detected, and reduce the output of at least the high frequency energy during the output reduction control period, before the probe is detached from a contact state, based on the prestored amount of mist which is different according to the shape of the probe.

## Description

### Technical Field

The present invention relates to an energy treatment system and a control method thereof, which perform treatment by outputting ultrasonic energy and high frequency energy in combination.

### Background Art

Generally, as a device for performing treatment such as incision or coagulation to a treatment subject site such as a body tissue, for example, Patent Literature 1: Japanese Patent No. 5465353 discloses an energy treatment instrument for use in a surgical system which performs treatment such as incision, in which a high frequency signal output (hereinafter referred to as a high frequency output or high frequency energy) and an ultrasonic signal output (hereinafter referred to as ultrasonic output or ultrasonic energy) are simultaneously output individually or in combination.

### Summary of Invention

The above-described energy treatment instrument can simultaneously perform incision and coagulation by simultaneously outputting ultrasonic energy and high frequency energy to a treatment subject site via a treatment portion provided at the tip, thereby performing treatment more smoothly. On the other hand, a small site scraped off from the treatment subject site by incision including fat, oil, and moisture diffuse into the air around the treatment portion in the form of mist. In the following description, a minute site, oil, and moisture diffused in a mist state are simply referred to as a mist.

In addition, a discharge (spark) occurs when a probe to which a high frequency output is applied comes into contact with a surgical instrument (for example, a treatment tool such as a forceps) used for surgery having a different potential and is then separated therefrom. When spark is applied under ambient environments in which many of minute sites diffuse into the air, that is, when three factors of the amount of mist, oxygen in the air, and the occurrence of spark are complete, an instantaneous local combustion phenomenon occurs. As a result, the view of the operator is blocked for a moment although it does not damage surrounding areas including the treatment site.

Depending on the surgical method, the occurrence of mist and the presence of air may be inevitable. Particularly, as disclosed in Japanese Patent No. 5465353, when the combination of the high frequency signal output and the ultrasonic signal output is used, the large amount of mist caused by the cavitation effect due to the ultrasonic signal output is generated. Although the occurrence of mist cannot be avoided, the combustion phenomenon can be avoided by preventing the occurrence of spark due to the high frequency output. Therefore, the occurrence of spark can be prevented by providing an interruption period in which the supply of the high frequency output is interrupted and stopped. On the other hand, since the treatment is performed only by the ultrasonic output during the interruption period, the incision performance of the energy treatment instrument is deteriorated and the surgeon may feel lack of the smoothness of the incision, thus feel a sense of incompatibility during the treatment.

Therefore, the present invention provides an energy treatment system and a control method thereof, which perform treatment by outputting a combination of ultrasonic energy and high frequency energy and perform control to detect a precursor of occurrence of spark during treatment.

According to an aspect of the present invention, an energy treatment system includes: an energy treatment instrument configured to perform treatment on a subject with a probe provided at a tip thereof by simultaneously outputting ultrasonic energy and high frequency energy in combination; a probe mist amount storage unit configured to store information about an amount of mist generated by the probe along with the ultrasonic energy; an ultrasonic energy generator configured to supply the ultrasonic energy to the energy treatment instrument; a high frequency energy generator configured to supply the high frequency energy to the energy treatment instrument; a system control unit configured to set an output reduction control period for reducing the output of the high frequency energy having a time length corresponding to the amount of mist, based on the information read from the probe mist amount storage unit; a spark occurrence precursor detection unit configured to detect a precursor of occurrence of spark by the probe or occurrence of spark by the probe; and an energy control unit configured to reduce at least the output of the high frequency energy during the output reduction control period based on information about the precursor or the occurrence of the spark from the spark occurrence precursor detection unit.

According to an aspect of the present invention, a method for controlling an energy treatment system includes: performing treatment on a subject by a probe of an energy treatment instrument which simultaneously outputs ultrasonic energy and high frequency energy in combination; setting an output reduction control period for reducing the output of the high frequency energy having a time length corresponding to an amount of mist, based on the prestored amount of mist which is different according to a shape of the probe; and reducing the output of at least the high frequency energy during the output reduction control period when a precursor of occurrence of spark by the probe is detected.

Brief Description of Drawings
FIG. 1 is a figure illustrating a conceptual configuration example of an energy treatment system according to a first embodiment.
FIG. 2 is a block diagram illustrating a configuration example of a short circuit detection unit.
FIG. 3 is a figure illustrating a state of change in a high frequency output voltage for explaining short circuit accompanying discharge.
FIG. 4 is a timing chart for explaining a first output control method.
FIG. 5 is a flowchart for explaining the first output control method.
FIG. 6 is a figure illustrating a conceptual configuration example of an energy treatment system according to a second embodiment.
FIG. 7 is a block diagram illustrating a configuration example of a short circuit detection unit according to the second embodiment.
FIG. 8 is a timing chart for explaining a second output control method.
FIG. 9 is a flowchart for explaining the second output control method.
FIG. 10 is a timing chart for explaining a third output control method according to a third embodiment.
FIG. 11 is a block diagram illustrating a configuration example of a short circuit detection unit according to a fourth embodiment.

### Description of Embodiments

Hereinafter, embodiments of the present invention will be described in detail with reference to the drawings.

### [First Embodiment]

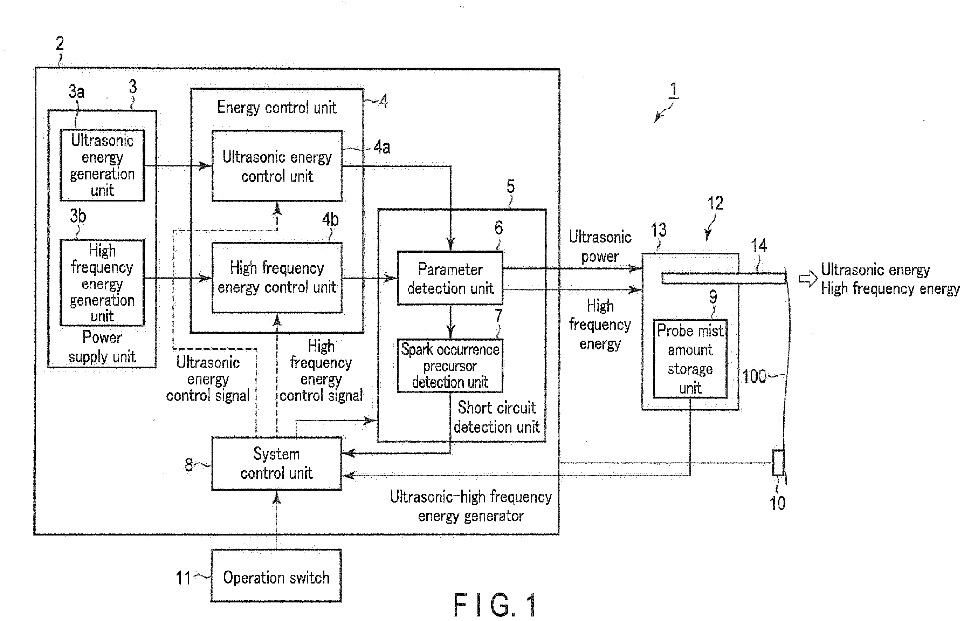

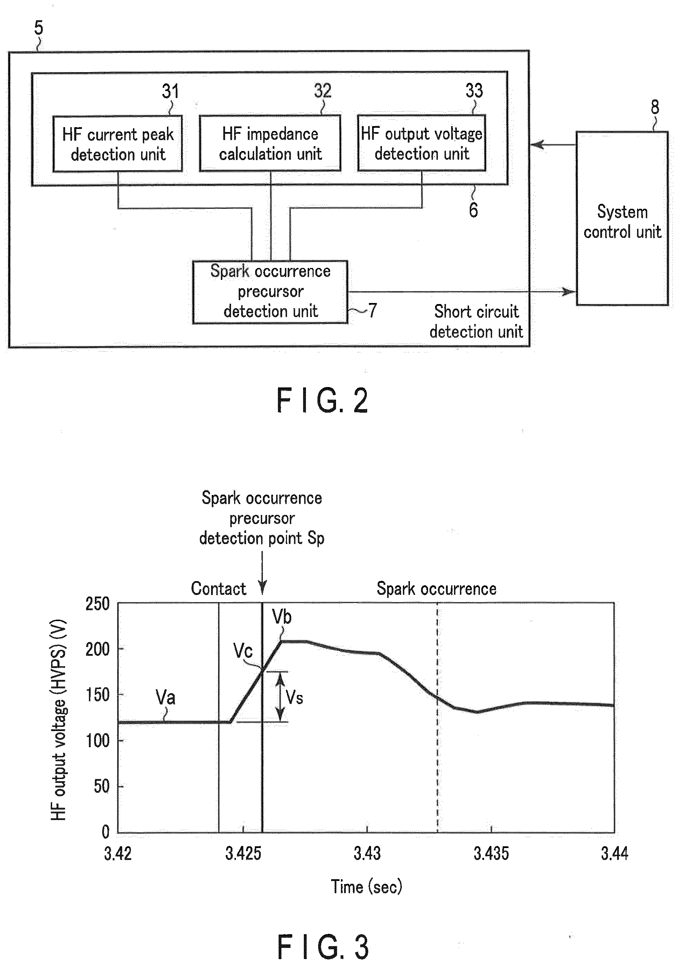

FIG. 1 is a figure illustrating a conceptual configuration example of an energy treatment system according to a first embodiment, and FIG. 2 is a figure illustrating a configuration example of a short circuit detection unit 5 according to the present embodiment.

The energy treatment system 1 includes an ultrasonic-high frequency energy generator 2, an operation switch 11, and an energy treatment instrument 12 such as an ultrasonic-high frequency combined handpiece equipped with an ultrasonic transducer (not shown).

The ultrasonic-high frequency energy generator 2 includes a power supply unit 3 which generates driving power including high frequency power, an energy control unit 4, a short circuit detection unit 5, a system control unit 8 including a central processing unit (CPU). This configuration shows only the components capable of realizing the technical features of the present invention, and other commonly provided components such as a display unit are normally provided. In the following description, energy includes electric energy, including high frequency power and other driving power, and vibration wave energy generated by vibration. In some cases, high frequency may be abbreviated as HF.

The power supply unit 3 includes an ultrasonic energy generation unit 3a which generates ultrasonic power for driving an ultrasonic transducer to generate ultrasonic energy, and a high frequency energy generation unit 3b which generates high frequency energy (high frequency output).

The energy control unit 4 includes an ultrasonic energy control unit 4a and a high frequency energy control unit 4b. The energy control unit 4 receives a high frequency energy control signal and an ultrasonic energy control signal, described later, from the system control unit 8. The high frequency energy control unit 4b performs the supply or stop of high frequency energy and performs the increase or decrease of the output value. The ultrasonic energy control unit 4a performs the supply or stop of ultrasonic power and performs frequency modulation. The energy control unit 4 may be not only a circuit configuration of a switching circuit or the like but also a software control, and has general versatility so that it is possible to appropriately select and execute the rise state of the high frequency energy control signal and the ultrasonic energy in a return period of each output control method described later according to the instruction of the system control unit 8.

The short circuit detection unit 5 includes a parameter detection unit 6 and a spark occurrence precursor detection unit 7. The parameter detection unit 6 detects or calculates a parameter used for detecting the precursor of occurrence of spark. The spark occurrence precursor detection unit 7 detects the presence or absence of the precursor of occurrence of spark and transmits each result to the system control unit 8. The precursor of occurrence of spark indicates the change of electrical and physical parameters occurring before the occurrence of spark.

The system control unit 8 has a function of performing driving control on the components in the ultrasonic-high frequency energy generator 2. Based on the result of the short circuit detection unit 5, the system control unit 8 maintains the normal outputs of the ultrasonic energy and the high frequency energy to a subject and continues the treatment, or prevents the occurrence of spark by continuing the treatment in a control state in which the output of the ultrasonic energy and the high frequency energy is reduced although the outputs thereof are not interrupted to prevent a local combustion phenomenon triggered by the spark. As described later, the energy control unit 4 controls the respective output levels of the high frequency energy and the ultrasonic energy and performs control such as re-outputting each again when a set period has elapsed.

The operation switch 11 is provided on the ultrasonic-high frequency energy generator 2 side or the energy treatment instrument 12 side and instructs driving of the ultrasonic-high frequency energy generator 2.

The energy treatment instrument 12 will be described.

The energy treatment instrument 12 is a configuration example of a monopolar type in which the probe 14 extends from a treatment instrument main body 13 to a tip side and is combined with a counter electrode plate (external electrode) 10. The treatment instrument main body 13 includes an ultrasonic transducer (not shown) and a probe mist amount storage unit 9 which stores the amount of mist generated for each probe.

The ultrasonic transducer generates ultrasonic vibration by supplying ultrasonic power from the ultrasonic energy control unit 4a and outputs the ultrasonic vibration as ultrasonic energy. The energy treatment instrument 12 may be a bipolar type in which a forceps structure is provided on the tip side of the probe 14. As the forceps structure, an opening/closing movable jaw is attached to the probe tip.

The parameter detection unit 6 and the spark occurrence precursor detection unit 7 of the short circuit detection unit 5 will be described with reference to FIG. 2.

The parameter detection unit 6 includes an HF current peak detection unit 31, an HF impedance calculation unit 32, and an HF output voltage detection unit 33.

The HF current peak detection unit 31 detects an HF current peak value in the high frequency energy output from the energy control unit 4. The HF output voltage detection unit 33 detects a high frequency output voltage (HVPS) from the high frequency energy. The HF impedance calculation unit 32 calculates a power value from a high frequency current value and a high frequency output voltage, and further calculates a high frequency impedance value from the power value. The spark occurrence precursor detection unit 7 determines whether there is a precursor of occurrence of spark.

The spark occurrence precursor detection unit 7 will be described with reference to FIG. 3.

As described above, when the ultrasonic energy and the high frequency energy are simultaneously output to a treatment subject site 100 and the treatment such as incision is performed, a part scraped off from the treatment subject site, oil, and moisture also contain a fatty part, become a minute site, oil, and moisture and diffuse like a mist. For example, when discharge (spark) is applied in an environment in which many mist-like minute sites, oil, and moisture diffuse in the air containing a combustion assisting gas such as oxygen, that is, when three factors of mist, air (combustion assisting gas such as oxygen), and spark occurrence are complete, an instantaneous localized combustion phenomenon occurs. As a result, the view of the operator is blocked for a moment although it does not damage surrounding areas including the treatment site.

In the present embodiment, when the treatment site causes the combustion phenomenon under the atmosphere of the air (combustion assisting gas) and the amount of mist diffuses, measures to prevent the occurrence of the spark are necessary.

When it is determined that the spark currently occurs from the detection result of either of the HF current peak detection unit 31, the HF impedance calculation unit 32, and the HF output voltage detection unit 33 in the parameter detection unit 6, the spark occurrence precursor detection unit 7 does not detect the occurrence precursor, immediately transmits the determination result to the system control unit 8, and performs control to lower the high frequency energy described later. On the other hand, when there is the amount of mist enough to cause the combustion phenomenon in the atmosphere of the air (oxygen: combustion assisting gas) at the treatment site, the precursor of the occurrence of the spark is detected and the occurrence thereof is prevented.

The spark occurrence precursor detection unit 7 will be described with reference to FIG. 3.

Generally, the spark occurs when the probe 14 outputting the high frequency energy is detached from the contact state in which the probe 14 comes into contact with a conductive surgical instrument having a low potential such as a forceps, or when the surgical instrument is detached from the contact state at the treatment site at which the electrical resistance is high (treatment site surrounded by fat or the like). FIG. 3 shows the HF output voltage (HVPS) of the output high frequency energy. When the probe 14 contacts other surgical instruments, that is, when a short circuit occurs, an overcurrent flows, resulting in that the high frequency current reaches its peak value. At this time, after the HF output voltage also rises to an overvoltage, it flattens temporarily and descends as the probe 14 and other surgical instruments separate. The spark occurs when detached from the contact state and extinguishes when separated by more than a certain distance. As synchronized with this, the HF impedance descends due to the contact and becomes a low impedance value, and when the probe 14 is detached from the contacted surgical instrument, the HF impedance rises and returns to converge to the original impedance value.

The determination of the detection of the precursor of the spark by using the parameters from the HF current peak detection unit 31, the HF impedance calculation unit 32, and the HF output voltage detection unit 33 in the parameter detection unit 6 shown in FIG. 2 will be described.

When the probe 14 contacts the conductive surgical instrument, as shown in FIG. 3, the HF output voltage detection unit 33 detects a voltage change that increases from a normal HF output voltage Va to an overvoltage Vb. In a case in which a voltage exceeds a predetermined determination reference voltage Vc during the rise, when the probe 14 is detached, a spark occurs. In this case, on the assumption that the spark will occur, the spark occurrence precursor detection unit 7 determines that there is the precursor of the occurrence of the spark. It is set as spark occurrence precursor detection point or spark occurrence detection point. The determination reference voltage [threshold] Vc is set based on a difference voltage Vs from the normal HF output voltage Va. Therefore, the determination reference voltage Vc is not a unique threshold value. When the HF output voltage Va changes, the voltage value of the determination reference voltage Vc also changes. The determination reference voltage Vc in the present embodiment can be determined within the range of 10 V to 50 V, and the set value is preferably about 25 V.

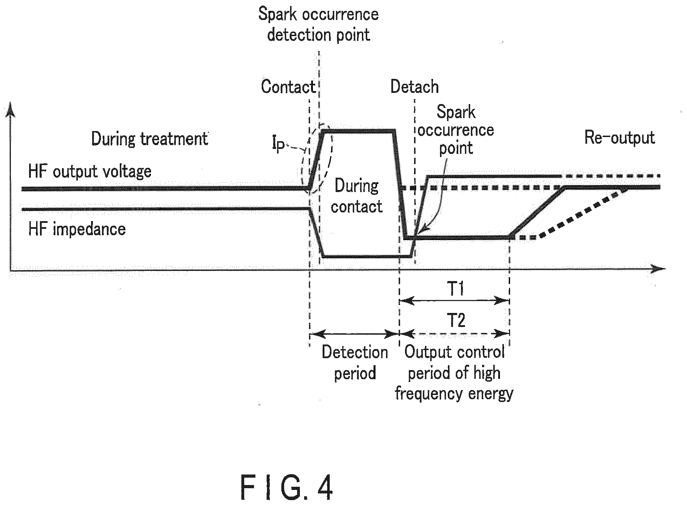

In addition, as shown in FIG. 4, the HF impedance calculation unit 32 detects decrease of the HF impedance when the probe 14 comes into contact with the conductive surgical instrument. In a case in which the decrease changes within the range of the preset determination reference impedance, when the probe 14 is separated from the contact state, the spark occurrence precursor detection unit 7 assumes that the spark will occur and determines that there is the precursor of the occurrence of the spark. The determination reference impedance can be determined with respect to the impedance value at the normal time before the contact. The decrease in the impedance value after contact may be within the range of -500 Ω to -1,500 Ω, and the set value is preferably about -1,000 Ω.

Furthermore, in a case in which an overcurrent flows when the probe 14 comes into contact with the conductive surgical instrument and a peak current thereof exceeds a preset change amount, the HF current peak detection unit 31 determines that it is the precursor of the occurrence of the spark during the separation of the probe 14 from the contact state. As for the change amount of the current, the measurement is performed for each device and set as appropriate.

The determination result of an occurrence factor determination unit 36 and the determination result of the spark occurrence precursor detection unit 7 are transmitted to the system control unit 8. From these determination results, the system control unit 8 select a control state. One state is a state in which the system control unit 8 continues usual treatment. The other state is a state in which the system control unit 8 continues the treatment in a state in which the output of the ultrasonic energy and the high frequency energy by the energy control unit 4 is lowered without stopping the treatment to prevent the occurrence of the combustion phenomenon triggered by the occurrence of spark.

In the present embodiment, under the environment in which the factors (mist amount and air) causing the above-described combustion phenomenon exist, the control to reduce the high frequency energy is performed by lowering the high frequency output before separation to prevent the occurrence of the spark causing the above-described combustion phenomenon when the probe 14 is separated from the conductive surgical instrument. The high frequency energy maintains the reduced state for at least the period as the output control period of the high frequency energy while being separated by a distance at which at least no spark reliably occurs. Even if the spark occurs, the spark may be extinguished before the combustion phenomenon occurs. Therefore, the output of the high frequency energy may be changed at any time, during the period from before the separation from the contact state to immediately after the separation, that is, during the period from before the occurrence of the spark to before the combustion phenomenon, after the probe 14 comes into contact with the conductive surgical instrument.

As an example, the lowered output of the high frequency energy (the high frequency voltage and current) is within the range of 10 W to 15 W, or is preferably about 10 W. The open circuit voltage is set to 300 Vrms or less, and the maximum high frequency current is set to 4 Arms or less. When an energy treatment system is actually manufactured, for example, the system configuration is limited to the open circuit voltage of 300 Vrms and the maximum high frequency current of 4 Arms.

In the present embodiment, the output reduction of the high frequency energy is set to start within at least 50 msec after determining the precursor of the occurrence of the spark. In addition, the output control period for reducing or stopping the output of the high frequency energy may be 20 msec or more, for example, 50 msec. In addition, as described later, when output restarts from the state in which the output is reduced or the output is stopped, it is preferable to gradually increase the output with a slope, for example, it is appropriately set within the range of 30 V/msec to 3 V/msec based on the shape of the probe and the mist generation amount.

It may be usually set to 15 W as the high frequency power which does not cause the combustion phenomenon. Although this example is an example in which the treatment is continued while reducing the high frequency energy to a value not causing the spark, the supply of the high frequency energy may be stopped.

The energy treatment instrument 12 will be described.

The energy treatment instrument 12 is a configuration example of a monopolar type in which the probe 14 extends from a treatment instrument main body 13 to a tip side and is combined with a counter electrode plate (external electrode) 10. The treatment instrument main body 13 includes an ultrasonic transducer (not shown) and a probe mist amount storage unit 9 which stores the amount of mist generated for each probe by ultrasonic vibration.

The ultrasonic transducer generates ultrasonic vibration by supplying ultrasonic power from the ultrasonic energy control unit 4a and outputs the ultrasonic vibration to the probe 14 as ultrasonic energy. The energy treatment instrument 12 may be a bipolar type in which a forceps structure is provided on the tip side of the probe 14. As the forceps structure, an opening/closing movable jaw is attached to the probe tip.

Since the ultrasonically vibrating probe is pressed against the treatment site and performs treatment so as to be scraped off, the amount of mist generated during the treatment with the probe 14 is affected by the magnitude of the level of cavitation generated at the probe 14 vibrating with ultrasonic energy. Therefore, it is possible to reduce the amount of mist generated by reducing the level of the cavitation.

Since the amount of mist generated at the time of the above-described treatment is affected by the magnitude of the level of the cavitation generated at the probe 14 vibrating with ultrasonic energy, the amount of generated mist can also be reduced by reducing the level of the cavitation. That is, although the efficiency of incision in surgery is reduced to some extent, the level of the cavitation is reduced by reducing ultrasonic energy, and the amount of mist generation also decreases. The generated mist floats, adheres to the vicinity, is discharged to the outside, and decreases with time.

The level of the cavitation is also greatly different in the shape of the treatment portion of the probe tip. In other words, even if the same ultrasonic vibration (ultrasonic energy) is supplied, the level of the cavitation decreases as the flat contact surface area decreases, in the treatment portion which is pressed against the treatment subject site 100, and the level of the cavitation increases as the protrusion such as a hook is provided, in the treatment portion. In other words, for example, as the treatment portion, the level of the cavitation increases and the amount of mist also increases in the order of a probe having a flat spatula, a probe having a curved spatula, and a probe having a protrusion such as a hook.

In the present embodiment, data about the magnitude of the level of the cavitation and the amount of mist generated when known ultrasonic energy is supplied to the probe 14 to be used is acquired. Any one of the magnitude of the level of the cavitation and the amount of mist generated may be acquired as long as the magnitude of the level of the cavitation and the amount of mist generated are related. In addition, when an operation to interrupt (stop) or reduce the supply of ultrasonic energy is assumed, a decrease time is acquired, the decrease time being a required time to reduce the amount of mist at which the combustion phenomenon does not occur. According to the amount of mist, the period of output reduction (or output interruption) of the high frequency energy is set so that the appropriate reduction interruption period can be set, and the timing of re-output is also set. Since the amount of mist generated is different according to the shape of the probe, the amount of mist generated at the level of the set cavitation (or ultrasonic energy) is acquired beforehand for all the probes having different shapes to be used, and the decrease time or the like is also acquired. As described later, the amount of mist acquired at this time is stored in the probe mist amount storage unit 9.

Therefore, as shown in FIG. 4, if the magnitude of the level of the cavitation is large, the amount of mist generated increases, the output control period (output reduction or output interruption period) of the high frequency energy becomes long, and the slope of the increase in re-output is gentle. When the slope of the above-described re-output is in the range of 30 V/msec to 3 V/msec, it is set to 3 V/msec. On the contrary, if the level of the cavitation is small, the amount of mist generated is reduced, the output control period of the high frequency energy is short, and the slope of the increase in re-output is set to 30 V/msec side.

The probe mist amount storage unit 9 prestores the amount of mist that is different according to the probe shape, and simply attaches the energy treatment instrument 12 to the ultrasonic-high frequency energy generator 2, so that it is possible to set the output reduction control period of the suitable high frequency energy. The amount of mist may be automatically set using ID information provided on the energy treatment instrument 12 side, the ID information being read by communication upon initial setting at the time of connection. A person who connects the probe 14 to the ultrasonic-high frequency energy generator 2 may set the amount of mist by selection instructions from various probes displayed on the screen. In addition, even if the energy treatment instrument 12 of another shape is changed during the treatment, it is possible to switch the output reduction control period of the high frequency energy only by reconnection.

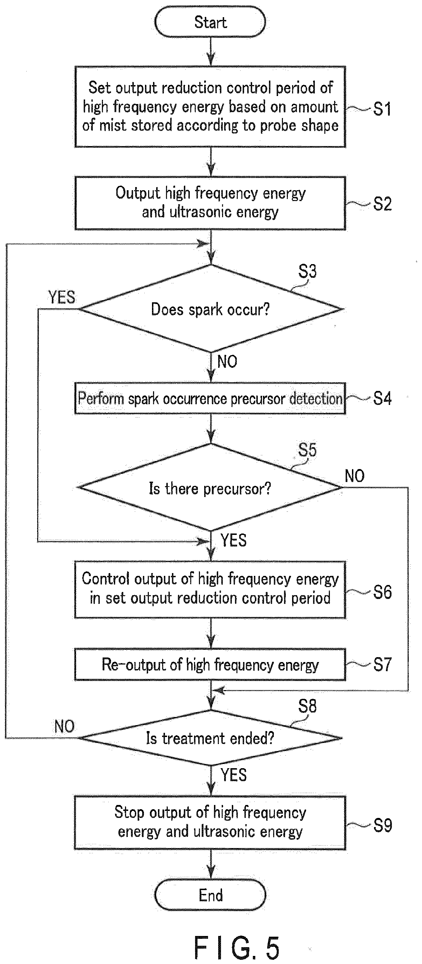

Next, the first output control method will be described with reference to the timing chart shown in FIG. 4 and the flowchart shown in FIG. 5.

First, when the energy treatment instrument 12 is connected to the ultrasonic-high frequency energy generator 2, the output reduction control period of the high frequency energy is set within the range of periods T1 to T2 based on the stored amount of mist (step S1).

Next, when the ON signal is input to the system control unit 8 by an ON operation of the operation switch 11, the power supply unit 3 is driven to generate ultrasonic energy and high frequency energy, and output the ultrasonic energy and the high frequency energy to the ultrasonic energy control unit 4a and the high frequency energy control unit 4b of the energy control unit 4, respectively.

The ultrasonic energy control unit 4a and the high frequency energy control unit 4b supply the ultrasonic energy and the high frequency energy, respectively, to the energy treatment instrument 12 via the short circuit detection unit 5 and simultaneously supply the ultrasonic energy and the high frequency energy to the tip of the probe 14. The energy treatment instrument 12 simultaneously outputs the high frequency energy and the ultrasonic energy, and the treatment of the treatment subject is performed (step S2).

According to the progress of the treatment, the living tissue containing the fat component included in the treatment subject site is generated in the mist state, and the mist increases around the probe 14. The mist includes the minute tissue fragment of the living tissue including the fat component scraped off by ultrasonic vibration and gas generated from the living tissue or adipose tissue burned by high frequency energy. The main proportion of the mist is assumed to be the minute tissue fragment of the living tissue containing the fat component and the moisture.

During the treatment, it is determined whether the spark occurs at present (step S3). In this determination, if the spark has occurred at present (YES), the determination result is immediately transmitted to the system control unit 8, and the output reduction control of the high frequency energy described later is performed. On the other hand, if no spark has occurred (NO), the spark occurrence precursor detection unit 7 detects the precursor of the occurrence of the spark (step S4).

The spark occurrence precursor detection unit 7 determines whether there is the precursor of the occurrence of the spark, within the detection period shown in FIG. 4, based on the detection results obtained from the HF current peak detection unit 31, the HF impedance calculation unit 32, and the HF output voltage detection unit 33 (step S5). In this determination, if there is no precursor of the occurrence of the spark (NO), the process proceeds to step S8 and the output of the high frequency energy is continued as it is while detecting the precursor. On the other hand, when it is determined that there is the precursor of the occurrence of the spark (YES), before the occurrence of the spark as shown in FIG. 4, or before leading to the combustion phenomenon from the spark, the output of the high frequency energy is reduced in the output reduction control period of the high frequency energy set based on the information read from the probe mist amount storage unit 9 and then set (step S6). At this time, the ultrasonic energy maintains the output, but the present invention is not limited thereto, and the output may be stopped according to the setting or circumstances (the length of the output control period or the like).

After that, from the state in which the output of the high frequency energy is reduced, the output is increased again with a slope and re-output (step S7). After the re-output, it is determined whether the treatment is to be continued (step S8). If the treatment is to be continued (NO), the process returns to step S3, and the detection is continued by the short circuit detection unit 5. On the other hand, if the treatment is ended (YES), the output of the high frequency energy and the ultrasonic energy is stopped (step S9), and the process is ended.

According to the present embodiment described above, when the treatment is performed with the probe which simultaneously outputs the high frequency energy and the ultrasonic energy, three occurrence factors (amount of mist, combustion assisting gas, and spark occurrence) which cause the combustion phenomenon are detected in the surrounding environment of the probe. In the present embodiment, the combustion assisting gas (oxygen) is assumed to be detected, when the treatment is performed in the condition where there is the atmosphere or the air around the probe, as the occurrence factor. A method of the detection of the presence or absence of the combustion assisting gas may be selected from a direct method where the presence or absence of the combustion assisting gas is detected and an indirect method where the surgical method (presence or absence of use of pneumoperitoneum described later) is set. These methods may be displayed on a display panel (not shown) or the like to be selected when the energy treatment instrument 12 is connected to the ultrasonic-high frequency energy generator 2.

The spark occurrence precursor detection unit 7 according to the present embodiment detects and determines the precursor of the occurrence of the spark, when the minute site containing the fat component, oil and moisture diffuse in the air containing the combustion assisting gas such as oxygen in the surrounding environment of the probe 14, and further become an environment having the spark occurrence factor. When the overcurrent or the overvoltage generated by the contact of the probe 14 exceeds a specified threshold value, the precursor spark precursor detection unit determines that the spark occurs. The precursor spark precursor detection unit reduces or interrupts the output of the high frequency energy before separation from the contact state of the probe 14, thereby preventing the occurrence of the spark and the occurrence of the concerned combustion phenomenon.

Furthermore, according to the present embodiment, it is possible to set a preferable output reduction control period of the high frequency energy based on the amount of mist derived from the ultrasonic vibration for each probe shape recorded in the energy treatment instrument. In other words, it is possible to prevent the occurrence risk of the combination of the ultrasonic vibration and the high frequency energy by changing and optimizing the output control parameter of the high frequency energy, which is an energy body different from the ultrasonic vibration, according to the parameters (amount of mist) for each probe recorded in the memory of the energy treatment instrument as the amount of mist based on the ultrasonic vibration.

In addition, since the amount of mist generated according to the shape of the probe can be grasped and the amount of generated mist can be assumed in advance, it is possible to grasp the presence or absence of the occurrence of the combustion phenomenon when the precursor of the occurrence of the spark is detected, and it is possible to set the control period of reducing the output of the high frequency energy to an appropriate output reduction control period which minimizes the reduction in the treatment efficiency.

### [Second Embodiment]

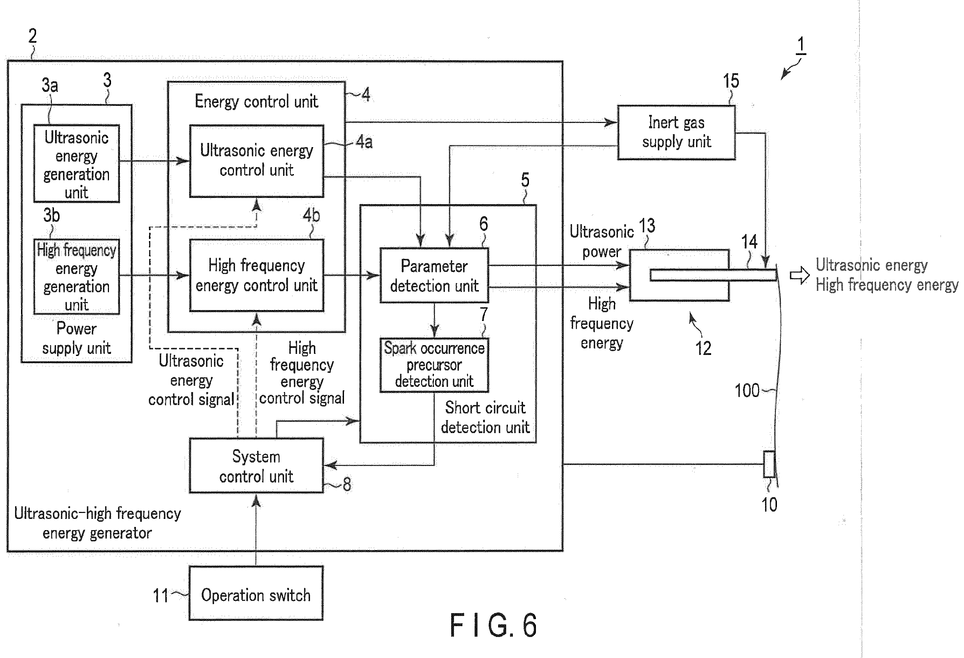

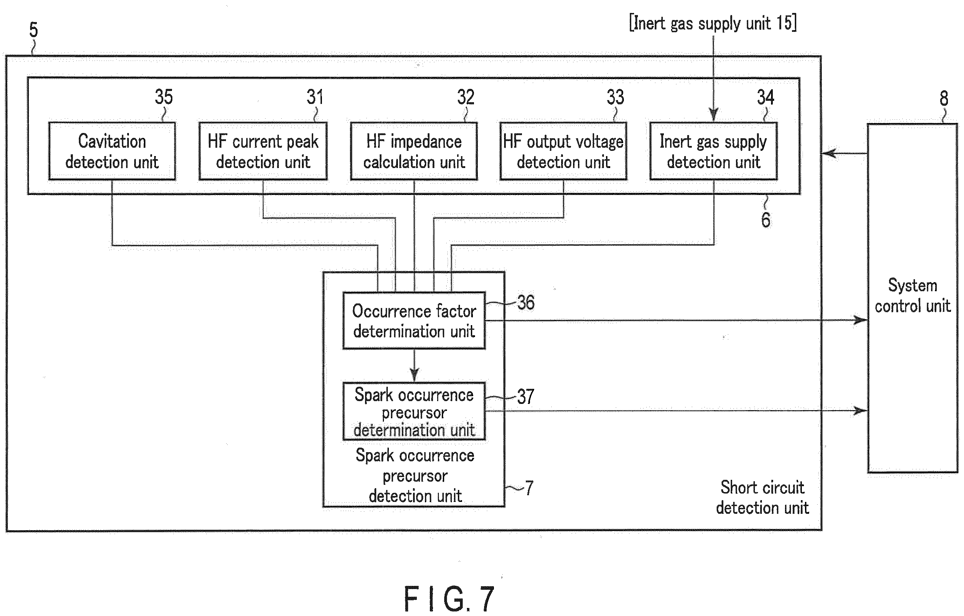

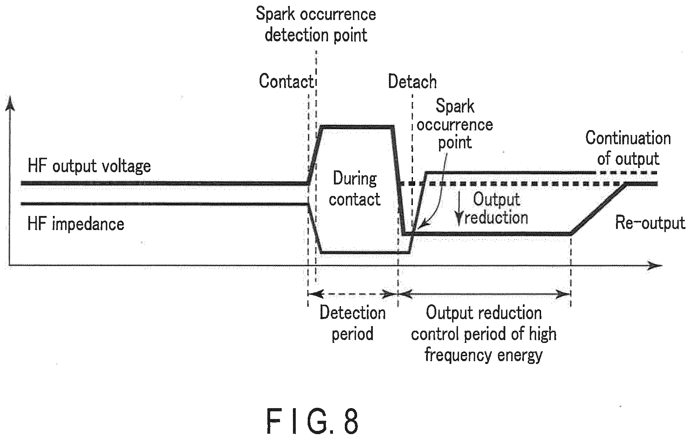

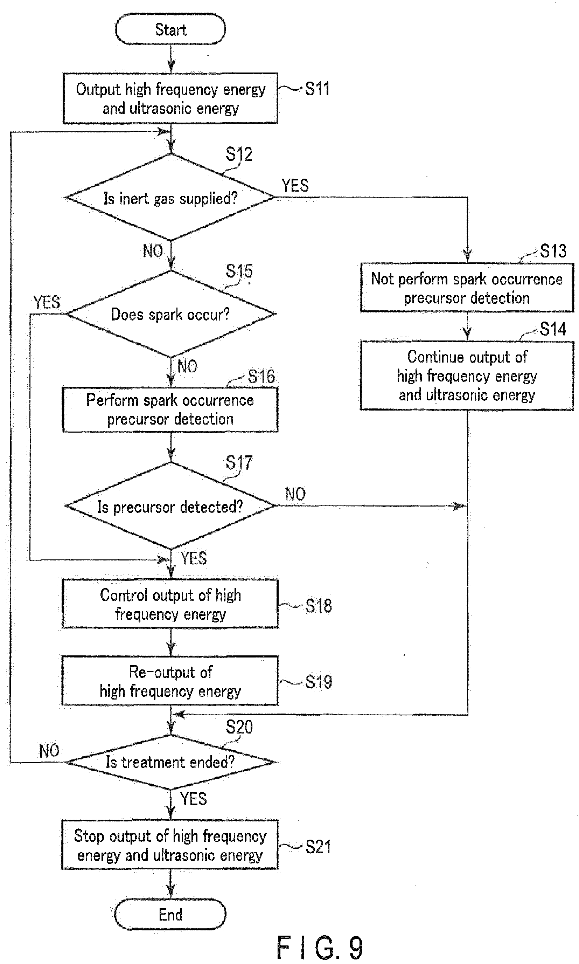

FIG. 6 is a figure illustrating a conceptual configuration example of an energy treatment system according to a second embodiment, and FIG. 7 is a block diagram illustrating a configuration example of a short circuit detection unit. FIG. 8 is a timing chart for explaining the second output control method, and FIG. 9 is a flowchart for explaining the second output control method. In the following description, the same components as those of the energy treatment system 1 in the first embodiment are denoted by the same reference numerals, the same step numbers are assigned to the same contents in the flowchart, and a detailed description thereof will be omitted.

The energy treatment system 1 includes an ultrasonic-high frequency energy generator 2, an operation switch 11, an energy treatment instrument 12 such as an ultrasonic-high frequency combined handpiece equipped with an ultrasonic transducer (not shown), and an inert gas supply unit 15.

The energy treatment system of the present embodiment includes the inert gas supply unit 15 which supplies an inert gas around the probe 14, and an inert gas supply detection unit 34 which detects that the inert gas is being supplied from the inert gas supply unit 15 (FIG. 7). When it is determined that the probe 14 is in an atmosphere of an inert gas, the occurrence factor determination unit 36 instructs the spark occurrence precursor detection unit 7 to interrupt the detection of the precursor of the occurrence of the spark.

Hereinafter, details will be described. The inert gas supply unit 15 shown in FIG. 6 supplies, for example, carbon dioxide (CO₂) gas to the vicinity of the probe 14 as the inert gas so as to secure the field of view during endoscope-based surgery.

Here, the combustion assisting gas and the inert gas in the treatment will be explained.

First, with respect to the combustion supporting gas, since the surgery involving incision such as laparotomy is performed under the atmosphere, it is impossible to exclude air containing the combustion supporting gas (oxygen). In the endoscope-based surgery in which a portal that is a small hole is opened in the vicinity of a treatment subject site and surgery is performed by inserting an endoscope such as a rigid endoscope or a treatment tool such as an energy treatment instrument, surgery is performed while supplying carbon dioxide gas by using, for example, a pneumoperitoneum as the inert gas supply unit 15 so as to secure the field of view. In this case, even if the spark occurs in the atmosphere in which there are many mists containing the fat component, the combustion phenomenon does not occur. As an assumption, even in surgery involving incision, if there is the form of blowing carbon dioxide gas around the probe outputting high frequency energy, it is also considered that the combustion phenomenon does not occur even if the spark occurs, as in the endoscope-based surgery.

The parameter detection unit 6 and the spark occurrence precursor detection unit 7 of the short circuit detection unit 5 of the present embodiment will be described with reference to FIG. 7.

The parameter detection unit 6 includes an HF current peak detection unit 31, an HF impedance calculation unit 32, an HF output voltage detection unit 33, an inert gas supply detection unit 34, and a cavitation detection unit 35. Among them, for example, the cavitation detection unit 35 may generate an electric signal from ultrasonic energy output from the ultrasonic energy control unit 4a by a detector using a piezoelectric body, and may set the electric signal as a detection signal. Furthermore, in the present embodiment, the cavitation may be associated with the amount of mist, and the cavitation may be detected from a known amount of mist in the probe 14 of the energy treatment instrument 12.

The inert gas supply detection unit 34 detects whether carbon dioxide gas is supplied from, for example, the inert gas supply unit 15 to the probe 14 when high frequency energy is applied, that is, during surgery.

The spark occurrence precursor detection unit 7 includes an occurrence factor determination unit 36 which determines whether to make a spark occurrence precursor determination, and a spark occurrence precursor determination unit 37 which determines whether there is the precursor of the occurrence of the spark.

The occurrence factor determination unit 36 determines the presence or absence of the above-described three factors causing the local combustion phenomenon, and if there is no combustion assisting gas, the occurrence factor determination unit 36 determines that no combustion phenomenon will occur even if the spark occurs. On the other hand, when a mist causing the combustion phenomenon in the atmosphere of the combustion assisting gas exists in the air at the treatment site, it is determined that it is necessary to prevent the occurrence of the spark. These determination results are instructed to the system control unit 8 and the spark occurrence precursor determination unit 37. Upon reception of the determination result of the occurrence factor determination unit 36, the spark occurrence precursor determination unit 37 determines whether to detect the precursor of the occurrence of the spark.

Therefore, in the determination as to whether the combustion assisting gas is present around the probe 14, for example, if the inert gas of carbon dioxide or the like is supplied around the probe, the occurrence factor determination unit 36 determines that no combustion phenomenon occurs, even if the amount of mist exceeds a specified value at which the combustion phenomenon occurs, and transmits the determination result to the system control unit 8. In response to the determination result, the system control unit 8 maintains the supply of the ultrasonic energy and the high frequency energy, which are the normal states so far. The instruction signal is also transmitted to the spark occurrence precursor detection unit 7 so as not to detect the precursor of the occurrence.

In addition to this determination, when it is determined that the spark currently occurs from the detection result of either of the HF current peak detection unit 31, the HF impedance calculation unit 32, and the HF output voltage detection unit 33 in the parameter detection unit 6, the occurrence factor determination unit 36 does not perform the detection of the occurrence precursor by the spark occurrence precursor detection unit 7, immediately transmits the determination result to the system control unit 8, and performs control to lower the high frequency energy described later.

On the other hand, in order to prevent the occurrence of spark when there is a mist causing a combustion phenomenon under an atmosphere of air (oxygen: combustion assisting gas) at the treatment site, the occurrence factor determination unit 36 transmits an instruction signal to the spark occurrence precursor detection unit 7 so as to detect the occurrence precursor.

The second output control method including spark occurrence precursor detection in the energy treatment system 1 in the embodiment will be described with reference to the timing chart shown in FIG. 8 and the flowchart shown in FIG. 9.

According to the ON operation of the operation switch 11, the system control unit 8 controls the ultrasonic energy control unit 4a and the high frequency energy control unit 4b, and supplies the ultrasonic power and the high frequency energy output from the power supply unit 3 to the energy treatment instrument 12. The ultrasonic energy and the high frequency energy are output. The energy treatment instrument 12 simultaneously outputs the high frequency energy and the ultrasonic energy to the tip of the probe 14, and the treatment of the treatment subject is performed (step S11). According to the progress of the treatment, the living tissue in the mist containing the fat component included in the treatment subject site is generated.

Next, from the detection result of the inert gas supply detection unit 34, the above-described occurrence factor determination unit 36 determines whether the treatment is performed under an environment in which an inert gas (carbon dioxide gas) is supplied (Step S12). In this determination, if the treatment is performed under the atmosphere of carbon dioxide gas (YES), it is determined that the above-described combustion phenomenon does not occur even if the spark occurs, no spark occurrence precursor detection is performed (step S13), and the output of the high frequency energy and the ultrasonic energy is continued as it is (step S14). While continuing the output, the process proceeds to step S20.

On the other hand, when it is determined that the treatment is not performed under the environment supplied with carbon dioxide gas (NO), the occurrence factor determination unit 36 further determines whether the spark occurs at present (step S15). In this determination, if the spark has occurred at present (YES), the determination result is immediately transmitted to the system control unit 8, and the control of the high frequency energy described later is performed. On the other hand, if no spark has occurred (NO), the precursor of the occurrence of the spark is detected by the spark occurrence precursor detection unit 7 (step S16).

The spark occurrence precursor detection unit 7 determines whether there is the precursor of the occurrence of the spark, within the detection period shown in FIG. 8, from the detection results obtained from the HF current peak detection unit 31, the HF impedance calculation unit 32, and the HF output voltage detection unit 33 (step S17). In this determination, if there is no precursor of the occurrence of the spark (NO), the output of the high frequency energy is continued as it is while detecting the precursor. On the other hand, when it is determined that there is the precursor of the occurrence of the spark (YES), before the occurrence of the spark as shown in FIG. 8, or before leading to the combustion phenomenon from the spark, the control of reducing the output of the high frequency energy is performed (step S18). At this time, the output reduction control period of the high frequency energy is appropriately set according to the probe shape, the amount of mist, or the cavitation as described above. In addition, in this example, the ultrasonic energy maintains the output, but the present invention is not limited thereto, and the output may be stopped according to the setting or circumstances (the length of the output control period or the like).

In the example shown in FIG. 3, if the output of the high frequency energy is reduced within about 10 msec (preferably within about 8 msec) as the time from the spark occurrence precursor detection point Sp prescribed by the applied current value or the overvoltage value to the spark occurrence, the occurrence of the combustion phenomenon due to the spark can be prevented. After the output control period, the output is increased with a slope and re-output from the state in which the output of the high frequency energy is reduced or the output is interrupted (step S19).

After the re-output, it is determined whether the treatment is to be continued (step S20). If the treatment is not to be ended (NO), the process returns to step 12, and the detection is continued by the short circuit detection unit 5. On the other hand, if the treatment is ended (YES), the output of the high frequency energy and the ultrasonic energy is stopped (step S21), and the process is ended.

According to the present embodiment described above, when the treatment is performed with the probe which simultaneously outputs the high frequency energy and the ultrasonic energy, three occurrence factors (amount of mist, combustion assisting gas, and spark occurrence) which cause the combustion phenomenon are detected in the surrounding environment of the probe. When the inert gas such as carbon dioxide is supplied around the probe and the atmosphere is in an inert gas atmosphere, the combustion phenomenon does not occur. Therefore, there is no need to detect the precursor of the occurrence of the spark and there is no need to reduce or interrupt the output of the high frequency energy.

The spark occurrence precursor detection unit 7 according to the present embodiment detects and determines the precursor of the occurrence of the spark, in the surrounding environment of the probe 14, when the minute site containing the fat component, oil and moisture diffuse in the air containing the combustion assisting gas such as oxygen and further become an environment having the spark occurrence factor. When the overcurrent or the overvoltage generated by the contact of the probe 14 exceeds a specified threshold value, the precursor spark precursor detection unit determines that the spark occurs and reduces or interrupts the output of the high frequency energy before separation from the contact state of the probe 14, thereby preventing the occurrence of the spark and the occurrence of the concerned combustion phenomenon.

### [Third Embodiment]

A third embodiment will be described below.

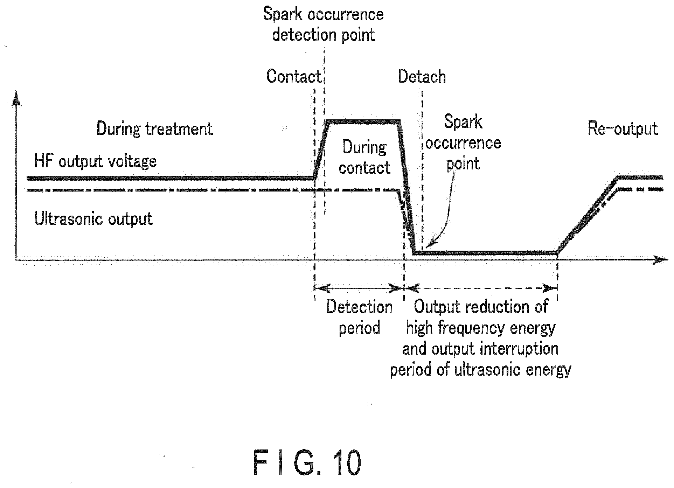

FIG. 10 is a timing chart for explaining a third output control method of an energy treatment system according to a third embodiment.

The present embodiment has the same configuration as the energy treatment system 1 of the first embodiment described above and will be described with reference to the device configuration shown in FIG. 1. The present embodiment is a control method for stopping the output of the ultrasonic energy, in addition to the output reduction of the high frequency energy or the output interruption (output stop) when the precursor of the occurrence of the spark is detected.

First, the ultrasonic power and the high frequency energy are output by the ON operation of the operation switch 11 under the control of the system control unit 8, and the treatment of the treatment subject is performed. From this progress, the mist increases around the probe 14. At this time, if it is determined that there is a possibility of combustion phenomenon, the detection of the precursor of the occurrence of the spark is performed by the spark occurrence precursor detection unit 7.

In a case in which there is contact between the probe 14 and another conductive surgical instrument, when it is determined that there is the precursor of the occurrence of the spark within the detection period after contact from the detection results obtained from the HF current peak detection unit 31, the HF impedance calculation unit 32, and the HF output voltage detection unit 33, the spark occurrence precursor detection unit 7 reduces the output of the high frequency energy to, for example, 15 W or less (preferably 10 W or less), without stopping the output of the high frequency energy, before the spark occurs or before the combustion phenomenon occurs from the spark. At the same time, the ultrasonic energy stops the output and waits until the amount of mist around the probe 14 decreases. The output reduction control period, which is the waiting time, is stored in the memory means in the above-described treatment instrument of the first embodiment, and is set based on the output reduction control period of the high frequency energy determined by the amount of mist set for each probe shape.

After the output control period, both the high frequency energy whose output is reduced and the ultrasonic energy whose output is stopped are re-output with a slope to increase the output.

According to the above-described present embodiment, in addition to the above-described first embodiment, when the precursor of the occurrence of the spark is detected, the output of the high frequency energy is reduced and the output of the ultrasonic energy is stopped. Therefore, the amount of mist around the probe quickly decreases, and consequently, the output reduction control period can be shortened.

In other words, it is possible to prevent the occurrence risk of the combination of the ultrasonic vibration and the high frequency energy by changing and optimizing the output control parameter of the high frequency energy, which is an energy body different from the ultrasonic vibration, according to the parameters (amount of mist) for each probe recorded in the memory of the energy treatment instrument as the amount of mist based on the ultrasonic vibration.

### [Fourth Embodiment]

A fourth embodiment will be described below.

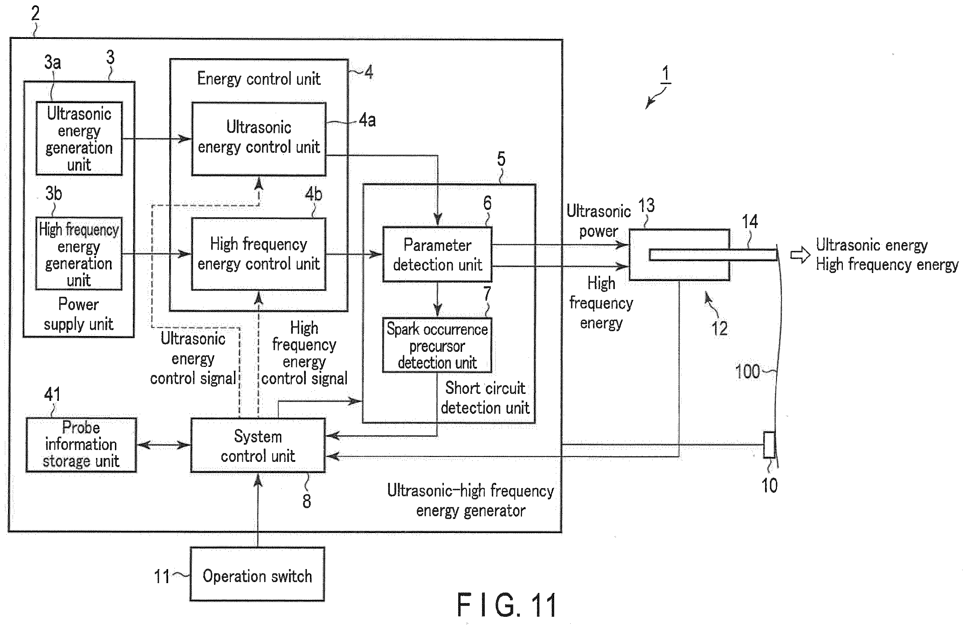

FIG. 11 is a block diagram illustrating a configuration example of a short circuit detection unit according to a fourth embodiment. In the following description, the same components as those of the energy treatment system 1 in the first embodiment are denoted by the same reference numerals, the same step numbers are assigned to the same contents in the flowchart, and a detailed description thereof will be omitted.

The above-described first embodiment is a configuration example in which the probe mist amount storage unit 9 for storing the amount of mist of the probe attached to the energy treatment instrument 12 is provided in the energy treatment instrument 12. Once the treatment is performed, the energy treatment instrument 12 may be discarded without processes such as sterilization for reuse. Therefore, in the present embodiment, in order to reduce the cost of the energy treatment instrument 12, the probe mist amount storage unit is provided as a probe information storage unit in the ultrasonic-high frequency energy generator 2, and the amount of mist for each probe and the output reduction control period of the high frequency energy set according to the amount of mist are stored.

With this configuration, when the energy treatment instrument 12 is connected to the ultrasonic-high frequency energy generator 2, a plurality of probes 14 having different shapes are displayed by a touch panel (not shown) or the like. A surgeon or a surgical staff selects the shape displayed in the same shape as that of the probe attached to the energy treatment instrument 12. The ultrasonic-high frequency energy generator 2 reads the output reduction control time of the high frequency energy from the probe information storage unit 41, and sets the read output reduction control time in the system control unit 8.

The probe information storage unit 41 stores the output reduction or the output interruption (output stop) of the ultrasonic energy as one piece of selection information, in addition to the information about the amount of mist and the output reduction control period of the high frequency energy. At the time of setting the probe shape, setting such as the reduction or the interruption of the output of the ultrasonic energy during the output reduction control period of the high frequency energy is performed. When the reduction or the interruption of the output of the ultrasonic energy is set, the time adjustment is further performed with respect to the output reduction control period of the high frequency energy selected according to the probe shape, taking into account the influence of the reduction or the interruption of the ultrasonic energy.

According to the present embodiment, it is possible to obtain the output reduction control period of the high frequency energy adjusted taking into account not only the output reduction control period of the high frequency energy according to the probe shape but also the output of the ultrasonic energy, and it is possible to more efficiently shorten the time to affect surgery with respect to the detected precursor of the occurrence of the spark.

In other words, it is possible to prevent the occurrence risk of the combination of the ultrasonic vibration and the high frequency energy by changing and optimizing the output control parameter of the high frequency energy, which is an energy body different from the ultrasonic vibration, according to the parameters (amount of mist) for each probe recorded in the memory of the energy treatment instrument as the amount of mist amount based on the ultrasonic vibration.

In addition, since the probe mist amount storage unit 9 is mounted on the energy treatment instrument 12 and is provided as a probe information storage unit in the ultrasonic-high frequency energy generator 2, an increase in manufacturing cost of the energy treatment instrument 12 can be suppressed.

## Claims

1. An energy treatment system comprising:
an energy treatment instrument configured to perform treatment on a subject with a probe provided at a tip thereof by simultaneously outputting ultrasonic energy and high frequency energy in combination;
a probe mist amount storage unit configured to store information about an amount of mist generated by the probe along with the ultrasonic energy;
an ultrasonic energy generator configured to supply the ultrasonic energy to the energy treatment instrument;
a high frequency energy generator configured to supply the high frequency energy to the energy treatment instrument;
a system control unit configured to set an output reduction control period for reducing output of the high frequency energy having a time length corresponding to the amount of mist, based on the information read from the probe mist amount storage unit;
a spark occurrence precursor detection unit configured to detect a precursor of occurrence of spark by the probe or occurrence of spark by the probe; and
an energy control unit configured to reduce at least the output of the high frequency energy during the output reduction control period based on information about the precursor or the occurrence of the spark from the spark occurrence precursor detection unit.

2. The energy treatment system according to claim 1, wherein the system control unit is configured to reduce the output of at least the high frequency energy among the ultrasonic energy and the high frequency energy during the output reduction control period, so that the spark is extinguished before the probe is detached from a contact state between the probe and a conductive surgical instrument, or immediately after the probe is detached from the contact state.

3. The energy treatment system according to claim 1, wherein the system control unit is configured to cause the energy control unit to perform re-output by increasing outputs of the high frequency energy with a slope after the output reduction control period.

4. The energy treatment system according to claim 2, wherein the energy control unit is configured to perform output reduction or output interruption of the ultrasonic energy during the output reduction control period for the output of the high frequency energy.

5. A method for controlling an energy treatment system comprising:
performing treatment on a subject by a probe of an energy treatment instrument which simultaneously outputs ultrasonic energy and high frequency energy in combination;
setting an output reduction control period for reducing output of the high frequency energy having a time length corresponding to an amount of mist, based on the prestored amount of mist which is different according to a shape of the probe; and
reducing the output of at least the high frequency energy during the output reduction control period when a precursor of occurrence of spark by the probe is detected.
